# EUROPEAN PATENT SPECIFICATION

(11) **EP 1 018 938 B1**
(45) Date of publication and mention of the grant of the patent: **05.12.2007**
(21) Application number: 98944192.8
(22) Date of filing: 10.09.1998
(51) Int. Cl.: A61B 5/083

(54) **BREATH TEST ANALYSER**
ATEMTEST ANALYSEVORRICHTUNG
ANALYSEUR DE TEST RESPIRATOIRE

(30) Priority: 11.09.1997 IL 12175197; 17.09.1997 IL 12179397
(43) Date of publication of application: 19.07.2000
(73) Proprietor: Oridion Breathid Ltd., Jerusalem 97774 (IL)
(72) Inventor: KATZMAN, Daniel, 99780 Kfar Bin Nun (IL); CARLEBACH, Ephraim, 43307 Raanana (IL)
(74) Representative: Hoarton, Lloyd Douglas Charles
(86) International application number: PCT/IL1998/000445
(87) International publication number: WO 1999/012471

(56) References cited:
- EP-A- 0 584 897
- EP-A- 0 672 383
- WO-A-97/14029
- US-A- 5 063 275

## Description

### FIELD OF THE INVENTION

The invention relates to the field of analyzers of the breath of patients to detect the gastric by-products of various diseases and infections.

### BACKGROUND OF THE INVENTION

Since the early 1950's, it has been known that the presence of bacterial organisms in the gastro-intestinal tract is accompanied by a high concentration of urease, which hydrolyses urea to form carbon dioxide and ammonia. These gases are detected in the subject's blood stream and ultimately, in the subject's breath, if he had been administered isotopically labeled urea. Such early results appear in reviews published by R.W. VonKorff et al in Am. J. Physiol., Vol. 165, pp. 688-694, 1951, and by H.L. Kornberg and R.E. Davies in Physiol. Rev., Vol. 35, pp.169-177, 1955.

Since these early experiments, it has been found that there exist, in addition to the bacterial infections initially studied, a significant number of medical conditions associated with disorders of the gastro-intestinal tract or metabolic or organ malfunctions, which are capable of detection by means of such simple breath tests. These breath tests are based on the ingestion of an isotopically labeled sample, which is cleaved by the specific bacteria or enzymic action being sought, or as a result of the metabolic function being tested, to produce labeled gaseous by-products. These by-products are absorbed in the blood stream, and are exhaled in the patient's breath, where they are detected by means of external instrumentation.

Though the early experiments were performed using the radioactive carbon-14 atom, the most commonly used atom in such test procedures today is the carbon-13 atom, which is a stable, non-radioactive isotope, present in a proportion of about 1.1% of naturally occurring carbon. The labeled substance contains the functional compound to be used in the test, with almost all of its ¹²C atoms replaced by ¹³C atoms. Enrichments of up to 99% of ¹³C are typically used. This compound is cleaved enzymatically under the specific conditions being tested for, either during gastric absorption, or during gastro-intestinal transit, or during its metabolisation in other organs of the body. The cleavage product produced is ¹³CO₂, which is absorbed in the bloodstream and exhaled in the patient's breath together with the CO₂ naturally present. The breath sample is then analyzed, usually in a mass spectrometer or a non-dispersive infra-red spectrometer. The increased presence of ¹³CO₂ is determined, as compared with the expected 1.1% of total CO₂ in a healthy patient's breath, resulting from the metabolism of carbon compounds with the naturally occurring level of approximately 1.1% of carbon-13.

Though carbon-13 is the most commonly used isotopic replacement atom in such breath tests, other atoms which have been used include nitrogen-15 and oxygen-18. In addition, carbon-14 is still used in some procedures, but being radioactive, there are severe disadvantages both to its ingestion by the patient, and because of the storage, handling and disposal precautions required at the test site.

There are an increasing number of metabolic disorders, bacterial infections and organ malfunctions which can be diagnosed using such labeled substances for enabling breath tests. New applications are being proposed continuously, but among the more common currently in use are:
(a) The detection of Helicobacter Pylori infections in the gastric and duodenal tracts, by means of the ingestion of ¹³C-labeled urea and breath detection of an increased level of ¹³CO₂. It is also feasible to use ¹⁵N-labeled urea, and to detect nitrogen-15 ammonia ¹⁵NH₃ in the breath, but this test format is not currently in use. Gastric and duodenal ulcers, non-ulcer dyspepsia and gastritis have been shown to be related to the presence of Helicobacter Pylori infections.
(b) The detection of fat malabsorption, such as is present in steatorrhea and Crohn's disease, by means of the ingestion of ¹³C-labeled triolein or tripalmitin, and breath detection of an increased level of ¹³CO₂.
(c) Liver function evaluation (by monitoring the P450 enzyme activity), liver disease severity and detoxification activity by means of the ingestion of ¹³C-labeled aminopyrin, methacitin or caffeine citrate (depending on the specific function being tested) and breath detection of an increased level of ¹³CO₂.
(d) The measurement of hepatic mitochondrial activity by means of the ingestion of ¹³C-labeled octanoic acid, and breath detection of an increased level of ¹³CO₂.
(e) A check of hepatic mitochondrial function efficiency by means of the ingestion of ¹³C-labeled ketoisocaproic acid, and breath detection of an increased level of ¹³CO₂.
(f) The quantification of functional liver mass by means of the ingestion of ¹³C-labeled galactose, and breath detection of an increased level of ¹³CO₂.
(g) The testing of gastric emptying function by means of the ingestion of ¹³C-labeled octanoic acid for the emptying rate of solids, or ¹³C-labeled sodium acetate for the emptying rate of liquids, and breath detection of an increased level of ¹³CO₂
(h) The determination of exocrine pancreatic insufficiency by means of the ingestion of a ¹³C-labeled mixed triglyceride sample such as octanoil-1,3-distearin for checking the lipase function, or a ¹³C-labeled sample of corn starch for checking the amylase function, and breath detection of an increased level of ¹³CO₂ The mixed triglyceride test is one of the tests used for detecting cystic fibrosis. For the evaluation of the digestion and absorption of medium-chain fatty acid triglycerides, ¹³C-labeled trioctanoin is used in preference to the mixed triglyceride.
(i) The detection of bacterial overgrowth in the small intestine by means of the ingestion of ¹³C-labeled glycolic acid or xylose, and breath detection of an increased level of ¹³CO₂.
(j) The testing of lactose or glucose intolerance, by means of the ingestion of ¹³C-labeled lactose or glucose, and measurement of the speed of appearance of an increased level of ¹³CO₂ in the breath.

Previously available tests for these illnesses generally involve drastically more invasive procedures, and are therefore much less patient compliant than the simple breath tests described above. Such procedures include gastro-endoscopy, with or without the removal of a tissue biopsy, biopsies of organs suspected of malfunction, blood tests to detect antibodies to suspected bacteria, blood biochemistry tests following ingestion of suitable compounds, and radiological tests, whether by gamma imaging of the organ function following ingestion or injection of a suitable gamma emitter, or by direct X-ray imaging or CT scanning. Furthermore, there are other disadvantages to the previously used tests, such as the fact that they rarely give real time information about the organ function or status being observed. In some cases, such as in the case of blood tests for antibodies of bacterial infections, they give historic results which may have no therapeutic relevance currently, since antibodies to a particular bacterium can remain in the body for up to 2 years from the date that the infection has been eradicated.

The above mentioned breath tests are completely non-invasive, and are executed in comparative real time, so that they have a great advantage over previously available tests, and their use is gaining popularity in the medical community, as evidenced by the fact that suitable isotopically labeled substances are currently available commercially from a number of sources.

However, in spite of the advantages of isotopically labeled breath tests, current instrumentation and procedures for performing it still have a number of serious drawbacks, which continue to limit its usefulness. The major disadvantage, which becomes apparent when a review of prior art breath test performance techniques and instrumentation is performed, is that none of the currently used techniques are sufficiently rapid to permit immediate measurement of the requested parameter, allowing a diagnosis for the patient in a single short visit to the physicians office.

One of the early breath tests to be proposed is that for detecting the presence of the Helicobacter Pylori bacterium in the upper gastro-intestinal tract, by means of the oral administration of isotopically labeled urea, and the detection of the presence of isotopically labeled carbon dioxide or ammonia in the patient's breath resulting from the hydrolysis of the urea by the urease which always accompanies H. Pylori infections. This method is described by Marshall in U.S. Patent No. 4,830,010. In this implementation of the test, the breath of the subject is collected, preferably from 10 to 120 minutes after administration of the substance, in a balloon inflated by the subject, and from there is transferred to a storage and transport container, such as a Vacutainer ® sold by Becton-Dickenson Inc.

According to a method proposed by Marshall, the sample is then analysed by mass spectrometry or by infra-red or nuclear magnetic resonance spectroscopy, for the presence of isotopically labelled CO₂ resulting from the hydrolysis of the urea. If the radioactive carbon-14 is used to label the urea, then the breath sample is analysed by bubbling it through a scintillation solution, which is transferred to a scintillation counter to determine the presence of beta radiation in the exhaled breath specimen. Because of the cost and complexity of the analysis instrumentation, in none of the preferred methods described by Marshall is it suggested that the analysis of the breath may be performed on site at the point where the sample is taken from the patient. The subject must thus wait at least ten minutes to give the sample, and must then wait for the laboratory to return the results. Clearly this method cannot be used to provide the results of the test within the context of a single visit to the office of the physician.

In a recent article entitled "Minimum Analysis Requirements for the Detection of Helicobacter pylori Infection by the 13C-Urea Breath Test" by P. D. Klieg and D. Y. Graham, published in Am. J. Gastroenterol., Vol. 88, pp. 1865-1869, 1993, a statistical study of the reliability and minimum criteria for conducting this test is presented. The breath analyses were again performed by gas isotope ratio mass spectrometry at a remote site. Amongst their findings are that breath sampling at 30 minutes after urea ingestion is likely to lead to significantly less false-positive and false-negative results, than sampling after 20 minutes, and that sampling after 30 minutes is therefore their proposed protocol time. They also conclude that "In the current environments of clinical research and patient care, the costs and turnaround times of CO₂ isotopic abundance measurements continue as the major barriers to commercial propagation of the ¹³C-urea breath test."

In another described prior art method of executing the urea breath test, Koletzko and co-workers describe the analysis of the exhaled breath by means of an isotope-selective non-dispersive infrared spectrometer [Koletzko et al., Lancet, 345:961-2, 1995]. Even using such a sophisticated instrument, the subjects are still required to wait 15 and 30 minutes for successive breath samples to be taken. Such a long delay to obtain breath samples, as well as the long wait between samples, is inconvenient and potentially reduces patient compliance.

Furthermore, as in the previously mentioned prior art, the sample or samples are collected from the patient and then sent to a laboratory for analysis, causing a delay in the determination of the results and forcing the subject to return to the office of the physician to obtain the results. If the test does not yield meaningful results, the entire process must be repeated again. The requirement for multiple office visits potentially further reduces patient compliance. The potential reduction in patient compliance can have serious consequences, since Helicobacter pylori is implicated by the World Health Organisation as a possible cause of stomach cancer, in addition to its role in gastric and duodenal ulcers.

The most rapid breath test currently proposed, the "Pytest" from Tri-Med Specialties, Charlottesville, N.C., USA, takes about 10-15 minutes to perform but uses radioactive carbon-14 isotopically-labeled urea [D.A.Peura, et al., Am. J. Gastro., 91:233-238, 1996]. The presence of ¹⁴CO₂ in the subject's exhaled breath is detected by direct beta counting. This test thus has all the disadvantages of the use of radioactive materials. Not only is the ingestion of radioactive materials potentially hazardous, but it also restricts the test to large testing centers which can handle such materials. Thus, the test cannot be performed in the office of the average physician, so that multiple office visits are again required.

Another recent prior art method which discusses implementations of the ¹³C-urea breath test, is shown in PCT Application No. WO97/14029, entitled "Method for Spectrometrically Measuring Isotopic Gas and Apparatus thereof, applied for by the Otsuka Pharmaceutical Company of Tokyo, Japan. In this application too, the exhaled breath sample is transferred in sample bags from the patient to the spectrometer, which, because of its cost, complexity and size, has perforce to be installed in a central sample collection laboratory, and not in the doctor's office or near the patient's bed. The inventors in fact state that "The measurement of such breath samples is typically performed in a professional manner in a measurement organisation, which manipulates a large amount of samples in a short time." This prior art proposes the use of one breath sample before the administration of the urea, and another after a lapse of 10 to 15 minutes.

Other prior art which describe sensitive analyzer systems for measuring the isotopic ratios of ¹³CO₂ to ¹²CO₂ in a gaseous sample, such as is required in an exhaled breath analyzer for performing the above mentioned breath tests, includes U. S. Patent No. 5,077,469, granted to W. Fabinski and G. Bernhardt, which describes a double reference path non-dispersive infra-red gas analyzer. A further development of such an instrument described in European Patent Application No. EP 0 584 897 A1 can be used to compare the two isotopic CO₂ concentrations in the exhaled breath by means of infra-red absorption measurements on two IR-cells filled with gas from the same breath sample.

In U.S. Patent Nos. 4,684,805 and RE 33493, granted to P.S. Lee, R.F. Majkowski and D.L.Partin, an infra-red absorbtion spectrometer is described for discriminating between the two isotopic CO₂ molecules for the breath tests. Their spectrometer design uses lead salt laser diodes as the source of radiation. Such laser diodes have emission lines in the 4µm to 5µm wavelength region of the infra-red spectrum, where the strongest CO₂ absorption lines are located. As a consequence, despite the lack of temperature stability of such laser diodes, and the fact that they must be operated at liquid nitrogen temperatures, their use enables the spectrometer to achieve the high selectivity and sensitivity required for breath test analysis.

U. S. Patent No. 5,317.156, granted to D.E. Cooper, C.B. Carlisle and H. Riris, describes an FMS (Frequency Modulation Spectroscopy) laser absorption spectrometer for distinguishing between the weak ¹²CO₂ and ¹³CO₂absorption lines in the 1.6 µm infra-red region, where highly stable laser diodes are available. Even though the CO₂ lines are very weak in this region, the stability of the GaAs laser diodes used as the source in this range, and the sophisticated TTFMS (two-tone Frequency Modulation Spectroscopy) technique used enables the inventors to provide sufficient differentiation between the two isotopes of CO₂ that the spectrometer can be used in breath test analysis.

In U.S. Patent No. 5,394,236, granted to D.E. Murnick, an apparatus for isotopic analysis of CO₂ is described by means of laser excited spectroscopy, utilising the optogalvanic effect to differentiate between the light of different wavelengths.

Because of the need to provide high sensitivity and good mass discrimination, all of the above described analysis systems are complex in nature. They are therefore, costly to manufacture and generally of large dimensions, making them suitable for commercial exploitation only for large and high sample volume installations.

A number of commercial companies offer complete systems for performing breath tests for the detection and study of the various gastro-enterologic conditions mentioned previously, using the isotopically labeled substances commercially available.

The Alimenterics Company of Morris Plains, New Jersey, markets the Pylori-Chek ¹³C-Urea breath test kit for use with its LARA^{™}System, for detecting the presence of H. Pylori in the gastro-intestinal tract. The company is developing kits for the clinical use of the other breath tests mentioned above. Breath is collected in a uniquely designed breath collection device, that also serves to transport the sample to the LARA^{™}System. This system, which stands for Laser Assisted Ratio Analyzer, is a sophisticated infra-red spectrometer designed to provide the sensitivity required to detect tiny percentage changes in the level of ¹³CO₂ in the patient's exhaled breath. Because of the complexity of the LARA^{™}System, it is a large piece of equipment, weighing over 300kg. and very costly. Consequently, this system too is only feasible for very large institutions and central laboratories, where the large number of tests performed can justify the cost.

Meretek Diagnostics Incorporated of Nashville, Tennessee, has also developed such a ¹³C-Urea breath test diagnostic system, and use an isotope ratio mass spectrometer called the ABCA (Automated Breath ¹³C Analyzer) manufactured by Europa Scientific Limited, of Crewe, Cheshire, U.K. for analyzing the breath samples. In this system too, the analyzer unit is large, costly and sophisticated, and therefore is usually located remote from the collection point.

Wagner Analysen Technik GmbH of Worpswede, Germany, offers an infra-red non-dispersive spectrophotometer-based system called the IRIS^{®} - Infra Red ISotope Analyser, which is based on the above-mentioned European Patent Application No. EP 0 584 897 A1. Though the main useage mode is by means of transport of the breath samples from the collection point to the analyzer in sample bags, this system, according to the manufacturer's sales literature, also has a sample port whereby connection can be made directly to a breathing mask, an incubator, or a breathing machine. No details of such a connection tube accessory are however given in the technical manual accompanying the analyzer, nor does the manufacturer provide any programs with the system's operational software to enable such an accessory to be used for performing on-line analyses. This analyzer has dimensions of 510 x 500 x 280 mm and weighs 12kg., and in addition, a PC is required for control. Though smaller and less costly than those mentioned above, it is still too large and heavy to be described as a truly portable device. Furthermore, its reported cost of several tens of thousands of U.S. Dollars, though considerably less than that of the two above-mentioned commercial systems, still makes it unsuitable for point-of-care or physician's office use.

In the preferred procedures described in all of the above mentioned prior art, the patient must wait typically 20 - 30 minutes before the active sample is collected, mainly because only one sample is taken beyond a background sample. This time is necessary to allow the level of isotopically labeled exhaled gas to reach a relatively high value, close to its end value, to enable the analyzer to measure the gas with a sufficient confidence level. However, such a single point determination potentially decreases the accuracy of the test, as well as increasing the risk of ambiguous results.

EP-A-0 672 383 discloses a method and apparatus for detection of venous ait emboli by continously measuring respiratory gases.

To the best of our knowledge, no breath test analyzer system has been described in the prior art which is sufficiently small, fast in producing reliable results, low in production cost, portable and sensitive, to enable it to be used as for executing tests in real time in the physician's office or at another point of care.

### SUMMARY OF THE INVENTION

The present invention seeks to provide an improved breath test analyzer which overcomes disadvantages and drawbacks of existing analyzers, which provides accurate results on-site in times of the order of minutes, and which is capable of implementation as a low cost, low volume and weight, portable instrument. The breath analyzer of the present invention is defined in the appended set of claims and is sufficiently sensitive to enable it to continuously collect and analyze multiple samples of the patient's breath from the beginning of the test, and process the outputs in real time, such that a definitive result is obtained within a short period of time, such as of the order of a few minutes.

Such a breath test analyzer is suitable for the detection of various disorders or infections of the gastro-intestinal tract, or metabolic or organ malfunctions, and since it can provide results in real time without the need to send the sample away to a special testing center or central laboratory, can be used to provide diagnostic information to the patient in the context of a single visit to a physician's office, or at any other point of care in a health care facility.

In accordance with a preferred embodiment of the present invention, there is provided a breath test analyzer, including a very sensitive gas analyzer, capable of measuring the ratio of two chemically identical gases but with different molecular weights, resulting from the replacement of at least one of the atoms of the gas with the same atom but of different isotopic value. Since the isotopically labeled gas to be measured in the patient's breath may be present only in very tiny quantities, and since, in general, it has an infra-red absorption spectrum very close to that of the non-isotopically labeled gas, the gas analyzer must be capable of very high selectivity and sensitivity, to detect and measure down to the order of a few parts per million of the host gas.

The breath test analyzer is also sufficiently small that it can easily be accomodated in the office of a physician, such as a gastro-enterologist, and its cost is also sufficiently low that its use in such an environment can be economically justified.

There are a number of different operational modes for each type of test for such a breath analyzer, the common denometer being that the analysis is performed in real time whilst the patient is continuing to provide breath for subsequent analyses. In the most common mode of operation, the breath test analyzer senses a patient's exhaled breath before ingestion of an isotopically labeled substance, analyzes the patient's exhaled breath for the percentage of the isotopically labeled gas in the total exhaled gas of that composition in order to obtain a baseline reading, performs at least one similar analysis after ingestion of an isotopically labeled substance, and provides an indication of a medical condition within a time period following the last sensing which is less than the difference in time between the first sensing of the patient's exhaled breath and the second sensing. This delineates it from previous breath analyzers, which, because of the generally remote location of the analyzer from the point at which the samples are given, cannot provide this indication within such a time limit.

In an alternative mode of operation, the analyses are made successively at times after ingestion of an isotopically labeled substance, and before the end of production of the isotopically labeled by-products of the substance, and the analyzer performs comparisons of the change from sample to sample of the percentage of the isotopically labeled gas in the total exhaled gas of that composition, and thereby provides an indication of a medical condition as soon as the detected change in gas composition percentage permits it, and before the end of production of the isotopically labeled by-products of the substance.

There are also two modes of analyzing the breath samples. The analyser can either perform its analysis on individual exhaled breaths, or, as stated above, it can perform its analysis on multiple samples of the patient's breath, continuously collected from the patient. The method of collection and subsequent analysis of multiple samples of the patient's breath has been described in Israel Patent Application No. 121793. That application describes an analyzer wherein the patient's breaths are exhaled into a resevoir for collection, in this application called a breath collection chamber, and transferred from there by one of various methods to the sample measurement chamber. One of the advantages of the method described therein, is that the analyzer draws an averaged sample of breath for measurement, instead of individual breaths, thereby increasing accuracy. Another advantage is that it is possible, by suitable valving means, to collect only the plateau parts of multiple breaths for analysis.

In accordance with a further preferred embodiment of the present invention, there is provided a breath test analyzer, which analyzes a first exhaled breath of a patient and a second exhaled breath of the patient for isotope labeled products generated in the patient's body after ingestion by the patient of an isotope labeled substance, by performing a first analyzing of the patient's first breath and a second analyzing of the patient's second breath, at least the second breath being exhaled following patient's ingesting the substance, the analyzer providing an indication of a medical condition within a time period following the exhalation of the second breath which is less than the difference in time between the exhalation of the first breath and the exhalation of the second breath.

There is further provided in accordance with yet another preferred embodiment of the present invention, a breath test analyzer as described above and including a breath analysis chamber, a breath inlet conduit for conveying exhaled gas from a patient to the breath analysis chamber; and a gas analyzer operative to analyze gas in the breath analysis chamber and to conduct the first analyzing of gas exhaled by the patient's first breath and the second analyzing of the patient's second breath, at least the second breath being exhaled following ingestion by the patient of an isotope labeled substance.

Furthermore, for those preferred embodiments which analyze samples collected from exhaled breaths of a patient, instead of individual breaths, it is understood that the analyzer also incorporates a breath collection chamber, which may be a separate chamber, or part of the breath inlet conduit., or part of the breath analysis chamber. In the latter case, the analysis of the gas sample effectively takes place in the breath collection chamber.

In accordance with another preferred embodiment of the present invention, there is provided a breath test analyzer as described above, and wherein the patient's first breath is exhaled prior to ingestation of an isotopically labeled substance, and the patient's second breath is exhaled following ingestation of the isotopically labeled substance

In accordance with yet another preferred embodiment of the present invention, there is provided a breath test analyzer as described above, and wherein both of the patient's first and second breaths are exhaled following patient's ingestation of the isotopically labeled substance.

There is further provided in accordance with another preferred embodiment of the present invention, a breath test analyzer which analyzes a patient's breath for isotope labeled products generated in the patient's body after ingestion by the patient of an isotope labeled substance, the analyzer providing an indication of a medical condition existent in the patient by analyzing at least two successive samples of the patient's breath, wherein the at least two successive samples of the patient's breath include at least one later sample exhaled following analysis of at least one earlier sample.

There is still further provided in accordance with another preferred embodiment of the present invention, a breath test analyzer as described above and including a breath analysis chamber, a breath inlet conduit for conveying exhaled gas from a patient to the breath analysis chamber, and a gas analyzer operative to analyze gas in the breath analysis chamber and to conduct analyses of the at least two successive samples of the patient's breath, wherein the at least two successive samples of the patient's breath include at least one later sample exhaled following analysis of at least one earlier sample.

In accordance with still another preferred embodiment of the present invention, there is provided a breath test analyzer which analyzes a patient's exhaled breath before and after a product of an isotope labeled substance ingested by the patient could be detected in the patient's breath, a first analyzing of the patient's exhaled breath taking place prior to the product being detectable in the patient's breath and a second analyzing of the patient's exhaled breath taking place once the product could be detectable in the patient's breath, the analyzer providing an indication of a medical condition within a time period following the exhalation of the second breath which is less than the difference in time between the exhalation of the first breath and the exhalation of the second breath.

There is further provided in accordance with other preferred embodiments of the present invention, a breath test analyzer which analyzes a first exhaled breath of a patient and a second exhaled breath of the patient for the products of an isotope labeled substance ingested by the patient while the patient is coupled to the device, or analyzes the above mentioned exhaled breath and provides an indication of a medical condition while the patient is coupled to the device, or is breathing into the device. The patient whose breath is being analyzed can be coupled to the device continuously from the analyzing of the first exhaled breath to the analyzing of the second exhaled breath.

There is still further provided in accordance with the present invention, a breath test analyzer as described above and including a breath analysis chamber, a breath inlet conduit for conveying exhaled gas from a patient to the breath analysis chamber, and a gas analyzer operative to analyze gas in the breath analysis chamber while the patient is coupled to the device.

There is even further provided in accordance with still another preferred embodiment of the present invention, a breath test analyzer as described above and including a breath analysis chamber, a breath inlet conduit for conveying exhaled gas from a patient to the breath analysis chamber, and a gas analyzer operative to analyze gas in the breath analysis chamber and to provide an indication of a medical condition while the patient is coupled to the device.

There is also provided in accordance with another preferred embodiment of the present invention, a breath test analyzer as described above and including a breath analysis chamber, a breath inlet conduit for conveying exhaled gas from a patient to the breath analysis chamber; and a gas analyzer operative to analyze gas in the breath analysis chamber and to provide an indication of a medical condition while the patient is breathing into the device.

In accordance with still another preferred embodiment of the present invention, there is provided a breath test analyzer as described above and wherein the patient is coupled to a disposable breath input device.

In accordance with yet another preferred embodiment of the present invention, there is provided a medical sample analyzer which analyzes samples taken from a patient, and wherein either the taking or the analyzing of the samples is terminated automatically at a point in time determined by the results of the analyzing of the samples.

In accordance with even another preferred embodiment of the present invention, there is further provided a breath test analyzer which analyzes samples of a patient's breath for isotope labeled products generated in the patient's body after ingestion by the patient of an isotope labeled substance, and wherein either the taking or the analyzing of the samples is terminated automatically at a point in time determined by the results of the analyzing of samples.

There is also provided in accordance with another preferred embodiment of the present invention, a medical sample analyzer as described above, which analyzes samples taken from a patient and including a sample input port for receiving samples taken from the patient and an analyzing apparatus for analyzing the samples, and wherein the analyzing is terminated automatically at a point in time determined by the results of the analyzing of the samples.

There is further provided in accordance with another preferred embodiment of the present invention, a breath test analyzer as described above and including a breath analysis chamber, a breath inlet conduit for conveying exhaled gas from a patient to the breath analysis chamber; and a gas analyzer operative to analyze gas in the breath analysis chamber and wherein the analyzing of samples from the patient is terminated automatically at a point in time determined by the results of the analyzing of the samples.

In accordance with another preferred embodiment of the present invention, there is further provided a breath test analyzer as described above, and wherein the gas analyzer includes a gas discharge tube gas analyzer, or an infra-red source which emits a discontinuous spectrum.

There is provided, in accordance with the invention as defined in the appended set of claims, a breath test analyzer as described above, and wherein the results of the analyzing of successive samples are fitted to a curve, and an indication of a medical condition in a patient is determined by inspecting the derivative of the curve. There is further provided, in accordance with the invention as defined in the appended set of claims, a method of breath testing which analyzes a first exhaled breath of a patient and a second exhaled breath of the patient for isotope labeled products generated in the patient's body after ingestion by the patient of an isotope labeled substance, and comprising the steps of performing a first analyzing of the patient's first breath, subsequently performing a second analyzing of the patient's second breath, at least the second breath being exhaled following the patient's ingesting the substance, and providing an indication of a medical condition within a time period following exhalation of the second breath which is less than the difference in time between exhalation of the first breath and exhalation of the second breath.

There is further provided another method of breath testing which analyzes a patient's exhaled breath for the product of an isotope labeled substance ingested by the patient, and comprising the steps of performing a first analyzing of the patient's exhaled breath prior to the product being detectable in the patient's breath, performing a second analyzing of the patient's exhaled breath once the product is detectable in the patient's breath, and providing an indication of a medical condition within a time period following the exhalation of the second breath which is less than the difference in time between the exhalation of the first breath and the exhalation of the second breath.

Furthermore, whereas all of the above mentioned preferred embodiments have been described for breath analyzers which analyze a first exhaled breath of a patient and a second exhaled breath of the patient, it is understood that the operation of these preferred embodiments are equally valid for breath analyzers which analyze a first sample collected from at least a first exhaled breath of a patient, and a second sample collected from at least a second exhaled breath of a patient.

### BRIEF DESCRIPTION OF THE DRAWINGS

The present invention will be understood and appreciated more fully from the following detailed description, taken in conjunction with the drawings, in which:
Fig. 1 is a schematic view of a breath analyzer constructed and operative in accordance with a preferred embodiment of the present invention, showing its main component parts including the breath inlet conduit and the breath analysis chamber.
Fig. 2A is a schematic view of a patient connected to the breath test analyzer by means of a nasal cannula, and shows the compact size of the analyzer, together with its associated laptop PC used for controlling the analyzer.
Fig. 2B is similar to Fig. 2A, except that the patient is connected to the analyzer by means of a blowing tube which he puts into his mouth when a sample breath is required.
Figs. 3A to 3D show schematically the various steps of a complete breath test cycle. The test cycle is shown being performed using a nasal cannula for the breath sampling, but the same procedure can be performed with the samples collected by means of a mouth tube.

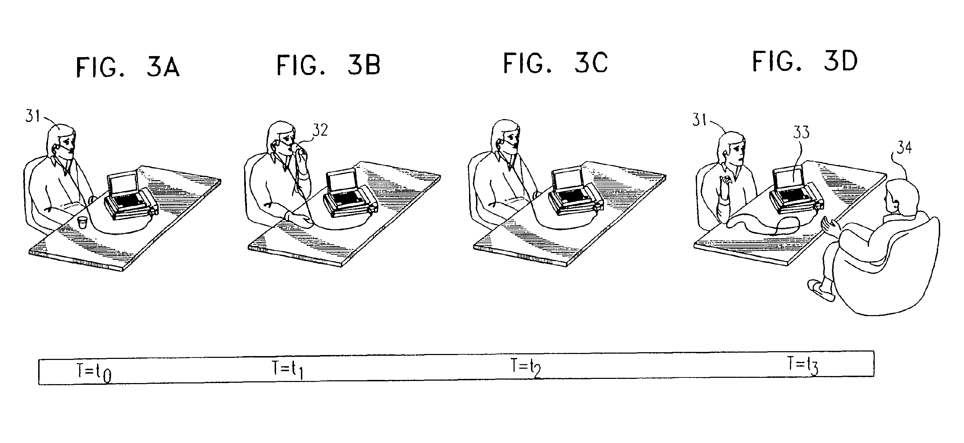

In Fig 3A, the patient is shown at time t₀ providing the reference breath before taking the isotopically labeled substance suitable for the specific test to be performed.
Fig. 3B shows the patient at time t₁ ingesting the isotopically labeled substance, shown in this instance in a glass of liquid.
Fig. 3C is a view of the patient at time t₂ providing continuous breath samples for the analyzer to collect through the nasal cannula or breathing tube. The analyzer itself measures the level of the isotopically labeled gas sample at regular intervals, and under the control of the PC, calculates the ratio of the isotopically labeled gas level to that of the naturally occuring gas of the same species for every breath sample, and subtracts the ratio from the baseline reference breath level. These ratios, known as the delta-over-baseline values, are fitted to a curve of ratio as a function of time, from which the results of the test can be deduced.
Fig. 3D shows the situation at time t₃ when the test has been completed and analysis terminated, either because the desired percentage level of the isotopically labeled gas has been reached, or because a time limit has been reached without a definitive delta-over-baseline percentage of gas having been reached. The PC is ready to show the results of the analysis prior to printout. Since the test is complete, the patient has removed the sampling device.

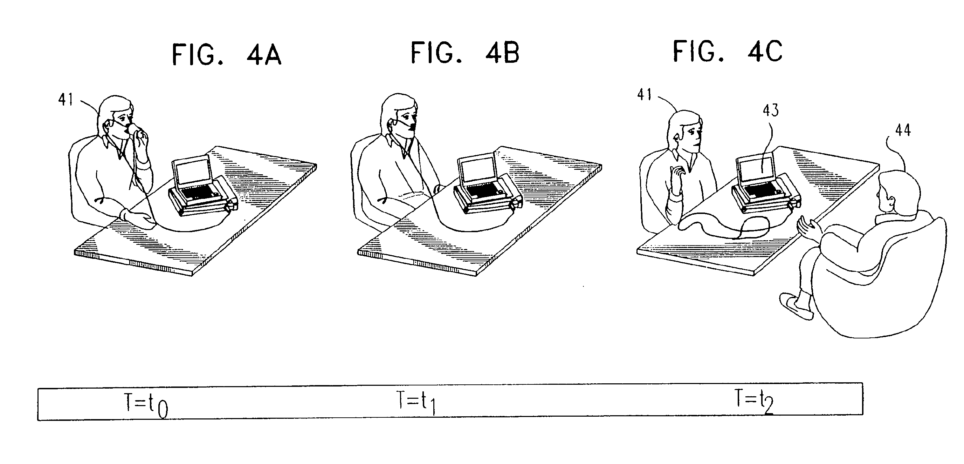

Figs. 4A to 4C show the various stages of a complete breath test cycle according to another preferred embodiment of the present invention, where the sampling analyses are performed at times following the ingestion of the isotopically labeled substance, without the need for a baseline measurement.
In Fig. 4A, the patient is shown at time t₀ ingesting the isotopically labeled substance, in this example in a glass of liquid.
In Fig. 4B, the patient is shown at time t₁ providing continuous breath samples for the analyzer to collect through the nasal cannula or breathing tube. The analyzer itself is measuring the level of the isotopically labeled gas sample at regular intervals, and under the control of the PC, is continuously calculating the ratio of the isotopically labeled gas level as compared to that of the previous measurement, in order to obtain a comparative reading of the change in the percentage level of the isotopically labeled gas from reading to reading as the breath test proceeds.
Fig. 4C shows the situation at time t₂ when the test has been completed and analysis terminated, either because the desired percentage increase in the level of the isotopically labeled gas has been reached, or because a time limit has been reached without a definitive percentage change having been detected. The display screen of the PC shows the results of the analysis prior to printout. Since the test is complete, the patient has removed the sampling device.

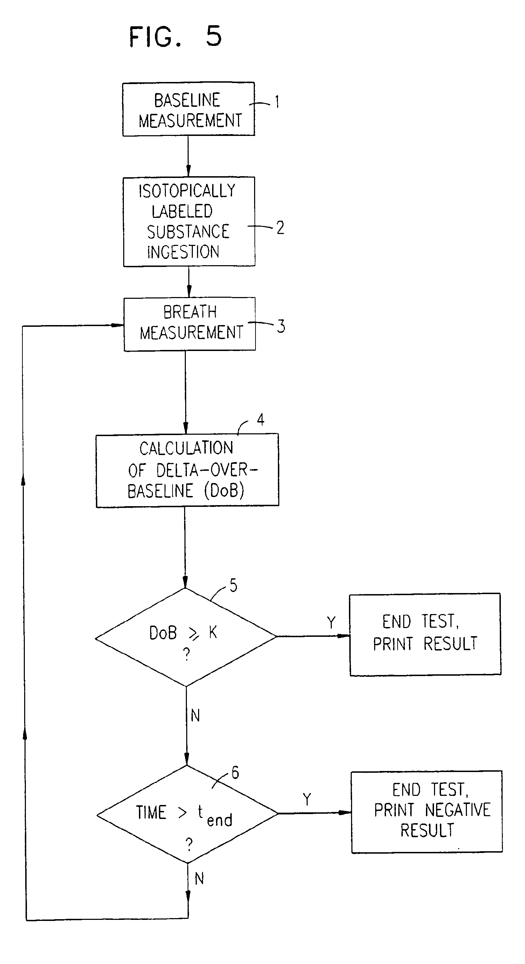

Fig. 5 is a schematic flow chart of the test procedures described in Figs. 3A to 3D, and in Figs. 4A to 4C.

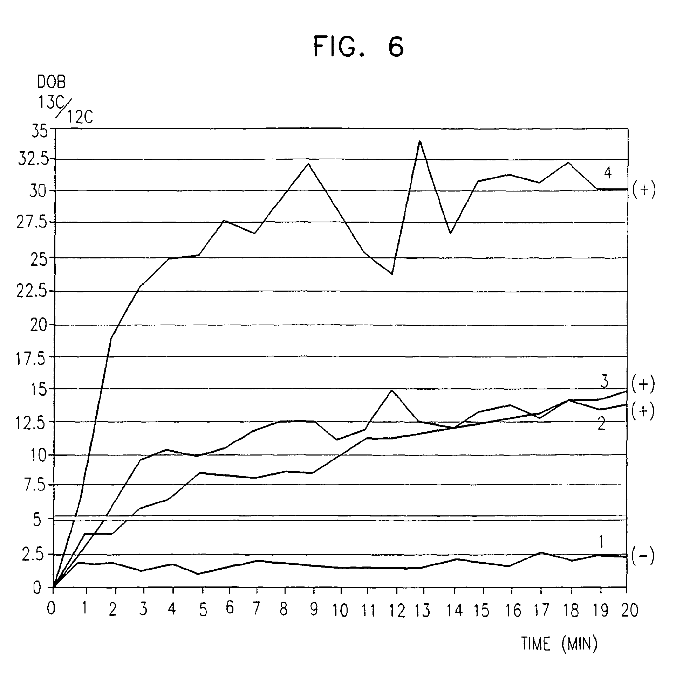

Fig. 6 shows a typical graph of the increase in ratio of the isotopically labeled gas as a function of time as the breath test proceeds, for a number of different patients.

### DETAILED DESCRIPTIONS OF PREFERRED EMBODIMENTS

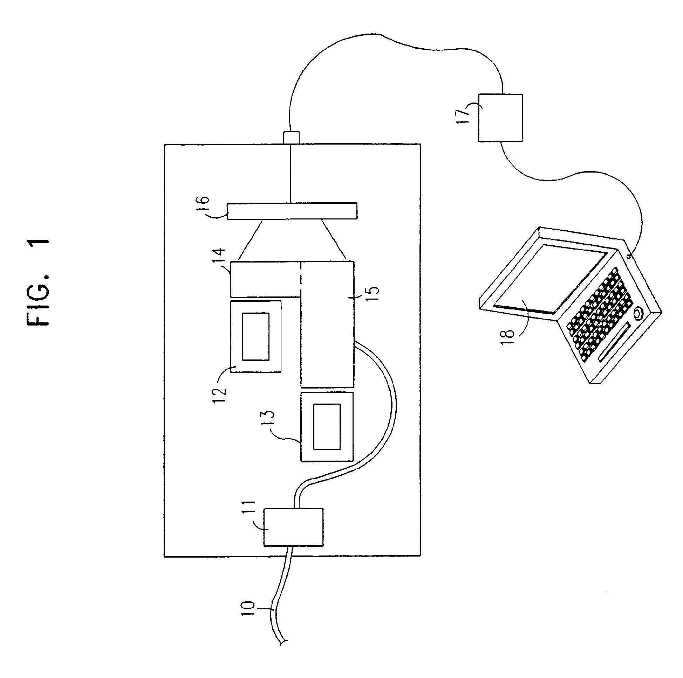

Reference is now made to Fig. 1, which is a schematic view of a compact high sensitivity breath analyzer constructed and operative in accordance with a preferred embodiment of the present invention. The breath analysis is performed by a sensitive non-dispersive infra-red spectrophotometer, capable of discriminating between the isotopically labeled CO₂ and the natural CO₂ in the breath sample being analyzed.

The patient is connected to the breath analyzer by means of the inlet tube 10, which can be either a nasal cannula or a breathing tube. Such a cannula includes a section of tubing, usually plastic, with two prongs. Each prong is inserted into a nostril and the cannula is then connected to the measuring instrument. As the patient exhales through the nose, a sample of the exhaled air flows through the cannula to the analyzer. A preferred type of breathing tube is constructed of a hollow tube held in the patient's mouth, through which he blows a number of breathes. In the center of the tube is located a small tube whose opening is positioned such that it samples the breath flowing through the main tube, and conveys it through a small flexible plastic inlet tube to the breath analyzer.

The patient's breath is inputted to the breath inlet conduit 11, which could also incorporate a breath collection chamber for acumulating a number of breaths, from where the breath sample is conveyed to the breath analysis chambers 14, 15 of a non-dispersive infra-red spectrophotometer. The breath analysis chamber could also be part of the breath collection chamber, such that the analysis is performed in the breath collection chamber. The spectrophotometer preferably uses gas discharge lamp sources 12, 13, such as those provided by Spegas Industries of Jerusalem, Israel. Such lamps enclose an enriched and nearly pure filling of ¹²CO₂ or ¹³CO₂ respectively. By excitation of an RF field, the gas discharge generates an emission which is typical of the CO₂ enclosed within the lamp. The average width of the emission lines from these lamps is only 0.006 cm⁻¹, such that there is little cross-sensitivity. It is possible to detect a change in isotopic gas concentration of the order of a few parts per million.

In order to obtain the ratio of ¹³CO₂/¹²CO₂ of a breath sample, the absorption of the sample is measured with a ¹²CO₂ lamp and a ¹³CO₂ lamp as light source. Such lamps have been used in a spectro-photometer described in U.S. Patent No. 5,063,275 incorporated herein by reference. The output signals are measured on an infra-red detector 16. The signals from this detector are processed electronically by the analyzer's electronics unit 17, and the resulting ratio output signal passed to the PC 18 for analysis by the system software according to the requirements of the measurement program.

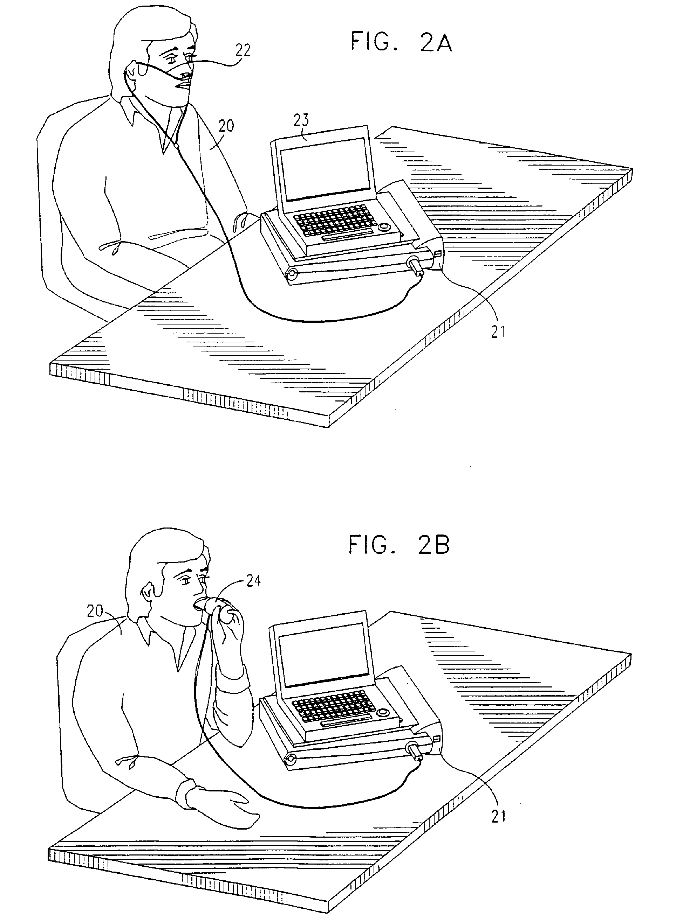

Fig. 2A is a schematic view of a patient 20 connected by means of a nasal cannula 22, to a breath test analyzer 21 constructed and operative according to a preferred embodiment of the present invention. A laptop PC 23 is used for controlling the analyzer. The compact size of the analyzer is apparent, when compared with the size of the laptop PC which stands on it. Fig. 2B is similar to Fig. 2A, except that the patient 20 is connected to the breath analyzer 21 by means of a blowing tube 24 which he puts into his mouth whenever sample breaths are required.

Figs. 3A to 3D show schematically the various steps of a complete breath test cycle in the most common mode of operation. The test cycle is shown being performed using a nasal cannula 30 for the breath sampling, but the same procedure can be performed with the samples collected by means of a mouth tube. In the most common mode of operation, the breath test analyzer senses a patient's breath before ingestion of an isotopically labeled substance, analyzes the patient's exhaled breath for the percentage of the isotopically labeled gas in the total exhaled gas of that composition in order to obtain a baseline reading, performs at least one similar analysis after ingestion of an isotopically labeled substance, and provides an indication of the increased presence of the isotopically labeled by-products characteristic of a medical condition, within a time period following the last sensing which is less than the difference in time between the first sensing and the last sensing. The analyses of the patient's exhaled breath may be performed directly, or on samples of exhaled breath collected in a breath collection chamber.

In Fig 3A, the patient 31 is shown at time t₀ providing the reference breath before taking the isotopically labeled substance suitable for the specific test to be performed. This reference breath enables the analyzer to establish a baseline level for the percentage of the isotopically labeled gas in the breath of the patient without the addition of any products of the isotopically labeled substance ingested.

Fig. 3B shows the patient at time t₁ drinking the isotopically labeled substance 32, shown in this instance in a glass of liquid.

Fig. 3C is a view of the patient at time t₂ providing continuous breath samples for the analyzer through the nasal cannula or breathing tube. The analyzer itself measures the level of the isotopically labeled gas sample at regular intervals, and under the control of the PC, calculates the ratio of the isotopically labeled gas level to that of the naturally occuring gas of the same species for every breath sample, and subtracts the ratio from the baseline reference breath level. These ratios, known as the delta-over-baseline values, are fitted to a curve of ratio as a function of time, from which the results of the test can be deduced. Each measurement takes a number of seconds, such that the analyses of the exhaled breath are effectively performed on a quasi-continuous basis. This is one of the main features which differentiates the procedure possible using a breath analyzer constructed and operative according to the present invention from all prior art procedures.

The technique proposed here, of performing a multiplicity of analyses or measurements under control of the measurement instrument itself, is applicable to a wide range of medical instrumentation. This technique allows the construction of an analyzer or measurement instrument, wherein the termination point of the test procedure being performed is determined automatically according to the results of the analyses or tests obtained in real time. The termination of the test procedure can refer not only to the termination of the taking of samples from the patient, but also to the termination of the analysis of such samples taken from the patient at an earlier time.

In the breath analyzer according to a preferred embodiment of the present invention, the multiplicity of analyses on substantially every successive breath, or on frequent samples of collected breaths, allows the analyzer to determine the termination point of the test procedure according to the results obtained in real time. In this most common mode of operation, the measurement system obtains for every breath sample, the ratio of the level of the isotopically labeled gas to that of the naturally occuring gas being analyzed. This ratio is then compared with the baseline ratio obtained at time t₀ in order to determine whether a positive result is being obtained. The delta-over-baseline level chosen to define a positive result is dependent on the specific test, and its sensitivity. The method of comparison of the measurement of one breath sample with the previous one can preferably be performed by means of fitting the results to a curve by one of the standard digital curve fitting methods, and determining the derivative of the curve at every new measurement point, or by simple repetitive difference measurements.

Fig. 3D shows the situation at time t₃ when the test has been completed and analysis terminated, either because the desired percentage increase in the level of the isotopically labeled gas has been reached, or because a time limit has been reached without a definitive delta-over-baseline percentage increase of gas having been reached. The display screen of the PC 33 shows the results of the analysis prior to printout. Since the test is complete, the patient 31 has removed the sampling device, and the patient's physician 32 is generally able to give him an immediate diagnosis, or at least the result of the test.

Figs. 4A to 4C show the various stages of a complete breath test cycle according to another preferred embodiment of the present invention, where the sampling analyses are performed at times following the ingestion of the isotopically labeled substance, without the need for a baseline measurement. This mode of operation is possible only because of the on-line nature of the measurements which the present invention enables. The method of comparison of the measurement of one breath sample with the previous one, can again be preferably performed by means of fitting the results to a curve by one of the standard digital curve fitting methods, and determining the derivative of the curve at every new measurement point.

In Fig. 4A, the patient 41 is shown at time t₀ ingesting the isotopically labeled substance, in this example in a glass of liquid.

In Fig. 4B, the patient is shown at time t₁ providing continuous breath samples for the analyzer to collect through the nasal cannula or breathing tube. The analyzer itself is measuring the level of the isotopically labeled gas sample at regular intervals, and under the control of the PC, is continuously calculating the ratio of the isotopically labeled gas level as compared to that of the previous measurement, in order to obtain a comparative reading of the change in the percentage level of the isotopically labeled gas from reading to reading as the breath test proceeds. In a preferred embodiment of the present invention, the analyzer program performs digital curve fitting analysis, as described above, in order to monitor the progress of the test.

Fig. 4C shows the situation at time t₂ when the test has been completed and analysis terminated, either because the desired percentage increase in the level of the isotopically labeled gas has been reached, or because a time limit has been reached without a definitive percentage change having been detected. The display screen of the PC 43 shows the results of the analysis prior to printout. Since the test is complete, the patient has removed the sampling device. As previously, the patient's physician 44 is able to advise him immediately of the result of the test.

The above mentioned operational modes of breath analyzing, and their methods of termination are functionally shown in the flow chart shown in Fig. 5, which is shown for the case when a baseline measurement is made before ingestion by the patient of the isotopically labeled substance. If no baseline measurement is made, the initial stage 1 of the flow chart is omitted, and in place of stage 4, an alternative calculation must be made, such as taking the difference between successive readings.

Fig. 6 shows graphs of the increase in ratio of the isotopically labeled gas as a function of time as the breath test proceeds, for a number of different patients. The actual results shown were obtained using a breath analyzer constructed and operative according to a preferred embodiment of the present invention, to detect ¹³CO₂ in the breath of patients after ingestion of ¹³C-labeled urea, for the detection of Helicobacter Pylori in the upper gastric tract. In the graphs shown, a value of 5 is chosen as the delta-over-baseline level to define a positive result. Patient number 1 thus has a negative result. Patients 2 and 3 show similar measurement curves, and it can be established after about 3 minutes that both of them have positive results. Patient number 4 has such a strong reaction to the ingest of the isotopically labeled substance that it becomes possible to provide a positive indication about his medical condition within 1 minute, and if the derivative method is used, in even less time.

The breath analyzer as proposed in the present invention is also operable in a number of different test modes, each with its own software package, for performing any breath test in which the patient ingests an isotopically labeled substance which produce isotopically labeled by-products detectable in the patients breath. Examples of a number of such breath tests are mentioned in the Background to the Invention section above.

It is clear that in all of the above preferred modes of operation, that the present invention provides a number of significant advantages over measurement procedures using previously available breath analyzers. Firstly, the exhaled breath of the subject can be analyzed in real time, so that there is relatively little delay between the time the specific gastro-intestinal reaction with the isotopically labeled substance takes place, and the time such activity is measured. Secondly, the samples of exhaled breath are obtained rapidly and are analyzed immediately in a manner which substantially increases the accuracy of the results. Thirdly, since multiple samples are obtained, the accuracy of the test is increased. Fourthly, there is less statistical error, since many samples are collected before a positive conclusion is reached. Fifthly, since samples are preferably collected until a preset level of accuracy is reached, ambiguous results can be substantially eliminated, preventing the need for repeat testing. Sixthly, since the analyzer itself makes the decision as to when sufficient samples have been analyzed to provide a clear indication of a medical condition, physiological differences between the response of different people to the various breath tests may be compensated for.

A further significant advantage of the use of the breath analyzer described in the present invention is that it increases patient compliance to a level that makes preventive medicine test procedures very acceptable. Furthermore, because of the considerably reduced costs of these tests, mass screening programs for a number of common gastro-enterological disorders could become more acceptable to health authorities and hence more widespread.

It will be appreciated by persons skilled in the art that the present invention is not limited by what has been particularly shown and described hereinabove.

## Claims

1. A method of breath testing comprising:
collecting at least a first sample from at least a first exhaled breath of a subject;
collecting at least a second sample from at least a second exhaled breath of the subject; and
analyzing at least said first sample and said second sample for products of an isotope labelled substance while the subject is coupled to a breath testing device with a detection sensitivity for said products of said isotope labelled substance in the order of a few parts per millions.

2. A method according to claim 1 and wherein said collecting at least a first sample, said collecting at least a second sample and said analyzing are performed continuously on multiple samples of the subject's breath.

3. A method according to any of the previous claims and also comprising the step of processing at least one output of said analyzing in real time.

4. A method according to any of the previous claims, wherein, said analyzing is performed in real time while the subject is continuing to provide breath for subsequent analyses.

5. A method according to any of the previous claims and wherein said analyzing of said first sample and said analyzing of said second sample are performed successively after ingestion of said isotopically labelled substance by the subject, and before the end of production of isotopically labelled by-products of said substance.

6. A method according to any of the previous claims and wherein said analyzing is performed on individual exhaled breaths.

7. A method according to any of the previous claims and wherein said collecting at least a first sample and said collecting at least a second sample are performed continuously.

8. A method according to any of the previous claims, and wherein said analyzing of at least one of said first sample and said second sample is performed on an accumulated number of breath samples collected from the subject.

9. A method according to any of the previous claims and wherein said collecting at least a first sample and said collecting at least a second sample are terminated automatically at a point in time determined by results of said analyzing.

10. A method according to any of the previous claims and wherein said analyzing is terminated automatically at a point in time determined by results of said analyzing.

11. A method according to any of the previous claims and also comprising measuring a level of isotopically labelled gas samples at regular intervals.

12. A method according to any of the previous claims and wherein said analyzing of said first sample and said analyzing of said second sample are effectively performed on a quasi-continuous basis.

13. A method according to any of the previous claims and wherein said at least a first sample and at least a second sample comprise a multiplicity of frequent samples of collected breaths, such that the termination point of the breath testing is determined according to the results of said analyzing obtained in real time.

14. A method according to any of the previous claims and wherein said at least a first sample and at least a second sample are collected from substantially every successive breath, such that the termination point of the breath testing is determined according to the results of said analyzing obtained in real time.

15. A method of breath testing according to any of the previous claims and wherein the results of said analyzing said first sample and said second sample are utilized to determine if a said testing has a clinically significant outcome.

16. A breath test analyzer comprising:
a breath analysis chamber,
a breath inlet conduit for-conveying exhaled gas from a subject to said breath analysis chamber, and
a gas analyzer operative to analyze gas in said breath analysis chamber, said breath test analyzer analyzing at least a first sample collected from at least a first exhaled breath of the subject and a second sample collected from at least a second exhaled breath of the subject for the products of an isotope labelled substance while the subject is coupled to said breath test analyzer, the said gas analyzer having a detection sensitivity for said products of said isotope labelled substance of the order of a few parts per million.

17. A breath test analyzer according to claim 16 and further comprising a breath collection chamber.

18. A breath test analyzer according to claim 17 wherein said breath analysis chamber, and said breath collection chamber are combined, such that said first and said second analyzing take place within said breath collection chamber.

19. A breath test analyzer according to any of claims 16-18, and which continuously collects and analyzes multiple samples of the subject's breath, and processes the outputs of said analysis in real time.

20. A breath test analyzer according to any of claims 16-19, and wherein said analysis is performed in real time while the subject is continuing to provide breath for subsequent analyses.

21. A breath test analyzer according to any of claims 16-19, wherein said analyses are made successively at times after ingestion of said isotopically labelled substance, and before the end of production of isotopically labelled'by-products of said substance.

22. A breath test analyzer according to any of claims 16-21 and which performs analysis on individual exhaled breaths.

23. A breath test analyzer according to any of claims 16-22 and which performs analysis of multiple samples of the subject's breath continuously collected from said subject.

24. A breath test analyzer according to any of claims 16-23 and wherein collection of said samples is terminated automatically at a point in time determined by results of analysis of said samples.

25. A breath test analyzer according to any of claims 16-24 and wherein said analysis of samples is terminated automatically at a point in time determined by results of said analyzing of said samples.

26. A breath test analyzer according to any of claims 16-25 and which measures a level of an isotopically labelled gas sample at regular intervals.

27. A breath test analyzer according to any of claims 16-26, and wherein analyses of the exhaled breath are effectively performed on a quasi-continuous basis.

28. A breath test analyzer according to any of claims 16-27 and which performs a multiplicity of analyses on substantially every successive breath, thereby allowing said analyzer to determine a termination point of a test procedure according to results obtained in real time.

29. A breath test analyzer according to any of claims 16-28 and which performs a multiplicity of-analyses on frequent samples of collected breaths, thereby allowing the analyzer to determine a termination point of a test procedure according to results obtained in real time.

30. A breath test analyzer according to any of claims 16-29, wherein said gas analyzer comprises a gas discharge tube gas analyzer.

31. A breath test analyzer according to any of claims 16-30, wherein said gas analyzer comprises an infrared source which emits a discontinuous spectrum.

32. A breath, test analyzer according to any of claims 16-31 and wherein said analyzer determines that a test has a clinically significant outcome in accordance with results of ongoing analyses of said first and second samples.

## Patentansprüche

1. Verfahren zur Atemtestung, umfassend
Abnahme mindestens einer Probe von mindestens einer ersten Ausatmung eines Probanden;
Abnahme mindestens einer zweiten Probe von mindestens einer zweiten Ausatmung des Probanden; und
Analysieren mindestens der ersten und der zweiten Probe auf Produkte einer durch Isotopen markierten Substanz, während der Proband an ein Atemtestgerät angeschlossen ist, mit einer Erkennungsemplindlichkeit für diese Produkte von isotopmarkierter Substanz in der Größenordnung einiger weniger Teile pro Million (ppm).

2. Verfahren gemäß Anspruch 1, bei dem die Abnahme mindestens einer ersten Probe, die Abnahme mindestens einer zweiten Probe und die Analyse kontinuierlich an einer Vielzahl von Atemproben des Probanden durchgeführt werden.

3. Verfahren gemäß einem der vorstehenden Ansprüche, das ferner den Schritt umfasst, mindestens ein Ergebnis der Analyse in Echtzeit zu verarbeiten.

4. Verfahren gemäß einem der vorstehenden Ansprüche, bei dem die Analyse in Echtzeit erfolgt, während der Proband weiter Atemgas für nachfolgende Analysen abgibt.

5. Verfahren gemäß einem der vorstehenden Ansprüche, bei dem die Analyse der ersten Probe und die Analyse der zweiten Probe sukzessive nach Aufnahme der isotopmarkierten Substanz durch den Probanden und vor Ende der Produktion isotopmarkierter Nebenprodukte dieser Substanz durchgeführt wird.

6. Verfahren gemäß einem der vorstehenden Ansprüche, bei dem die Analyse an einzelnen Ausatmungen erfolgt.

7. Verfahren gemäß einem der vorstehenden Ansprüche, bei dem die Abnahme mindestens einer ersten Probe und die Abnahme mindestens einer zweiten Probe kontinuierlich erfolgen.

8. Verfahren gemäß einem der vorstehenden Ansprüche, bei dem die Analyse von mindestens einer der ersten oder der zweiten Probe anhand einer kumulierten Anzahl von Atemproben erfolgt, die von dem Probanden genommen wurden.

9. Verfahren gemäß einem der vorstehenden Ansprüche, bei dem die Abnahme mindestens einer ersten Probe und die Abnahme mindestens einer zweiten Probe zu einem Zeitpunkt automatisch beendet werden, der durch Ergebnisse der Analyse bestimmt wird.

10. Verfahren gemäß einem der vorstehenden Ansprüche, bei dem die Analyse zu einem Zeitpunkt automatisch beendet wird, der durch Ergebnisse der Analyse bestimmt wird.

11. Verfahren gemäß einem der vorstehenden Ansprüche, ferner umfassend das Messen eines Niveaus isotopmarkierter Gasprohen in regelmäßigen Abständen.

12. Verfahren gemäß einem der vorstehenden Ansprüche, bei dem die Analyse der ersten Probe und die Analyse der zweiten Probe effektiv auf quasi-kontinuierlicher Basis durchgeführt werden.

13. Verfahren gemäß einem der vorstehenden Ansprüche, bei dem die mindestens eine erste Probe und die mindestens eine zweite Probe eine Vielzahl häufiger Probennahmen der Atemluft umfassen, sodass der Endpunkt des Atemtests entsprechend den in Echtzeit erzielten Ergebnissen der Analyse bestimmt wird.

14. Verfahren gemäß einem der vorstehenden Ansprüche, bei dem die mindestens eine erste Probe und mindestens eine zweite Probe von im Wesentlichen jedem folgenden Atemzug genommen werden, sodass der Endpunkt des Atemtests entsprechend den in Echtzeit erzielten Ergebnissen der Analyse bestimmt wird.

15. Verfahren gemäß einem der vorstehenden Ansprüche, bei dem die Ergebnisse der Analyse der ersten Probe und der zweiten Probe verwendet werden, um zu bestimmen, ob ein Test ein klinisch signifikantes Ergebnis liefert.

16. Atemlest-Analysator, umfassend:
eine Atemgas-Analysekammer;
eine Atemgas-Einlassleitung zum Transportieren des von einem Probanden ausgeatmeten Atemgases in die Atemgas-Analysekammer und
einen Gasanalysator, der dazu dient, Gas in der Atemgas-Analysekammer zu analysieren, wobei der Atemtest-Analysator mindestens eine erste Probe von mindestens einer ersten Ausatmung des Probanden und eine zweite, von mindestens einer zweiten Ausatmung des Probanden entnommene Probe auf die Produkte einer durch Isotopen markierten Substanz analysiert, während der Proband an den Atemtest-Analysator angeschlossen ist, wobei der Gasanalysator eine Erkennungsempfindlichkeit für die Produkte dieser isotopmarkierten Substanz in der Größenordnung einiger weniger Teile pro Million (ppm) aufweist.

17. Atemtest-Analysator gemäß Anspruch 16, ferner umfassend eine Atemgas-Sammelkammer.

18. Atemtest-Analysator gemäß Anspruch 17, bei dem die Atemgas-Analysekammer und die Atemgas-Sammelkammer kombiniert sind, sodass die erste und die zweite Analyse innerhalb der Atemgas-Sammelkammer erfolgen.

19. Atemtest-Analysator gemäß einem der Ansprüche 16-18, der kontinuierlich eine Vielzahl von Atemproben des Probanden nimmt und analysiert und die Ergebnisse dieser Analysen in Echtzeit verarbeitet.

20. Atemtest-Analysator gemäß einem der Ansprüche 16-19, bei dem die Analyse in Echtzeit erfolgt, während der Proband weiter Atemgas für nachfolgende Analysen abgibt.

21. Atemtest-Analysator gemäß einem der Ansprüche 16-19, bei dem die Analysen sukzessive zeitweise nach Aufnahme der isotopmarkierten Substanz und vor Ende der Produktion isotopmarkierter Nebenprodukte dieser Substanz durchgeführt wird.

22. Atemtest-Analysator gemäß einem der Ansprüche 16-21, bei dem die Analyse an einzelnen Ausatmungen durchgeführt wird.

23. Atemtest-Analysator gemäß einem der Ansprüche 16-22, bei dem eine Analyse einer Vielzahl von Atemproben des Probanden erfolgt, die kontinuierlich von dem Probanden genommen wurden.

24. Atemtest-Analysator gemäß einem der Ansprüche 16-23, bei dem die Abnahme der Proben zu einem Zeitpunkt automatisch beendet wird, der durch Ergebnisse der Analyse der Proben bestimmt wird.

25. Atemtest-Analysator gemäß einem der Ansprüche 16-24, bei dem die Analyse der Proben zu einem Zeitpunkt automatisch beendet wird, der durch Ergebnisse der Analyse der Proben bestimmt wird.

26. Atemtest-Analysator gemäß einem der Ansprüche 16-25, bei dem ein Niveau einer isotopmarkierten Gasprobe in regelmäßigen Abständen gemessen wird.

27. Atemtest-Analysator gemäß einem der Ansprüche 16-26, bei dem Analysen der Ausatmung effektiv auf quasi-kontinuierlicher Basis durchgeführt werden.

28. Atemtest-Analysator gemäß einem der Ansprüche 16-27, bei dem eine Vielzahl von Analysen von im Wesentlichen jedem folgenden Atemzug durchgeführt werden, sodass der Analysator einen Endpunkt einer Testprozedur entsprechend den in Echtzeit erzielten Ergebnissen bestimmen kann.

29. Atemtest-Analysator gemäß einem der Ansprüche 16-28, bei dem eine Vielzahl von Analysen von häufigen abgenommenen Atemproben durchgeführt werden, sodass der Analysator einen Endpunkt einer Testprozedur entsprechend den in Echtzeit erzielten Ergebnissen bestimmen kann.

30. Atemtest-Analysator gemäß einem der Ansprüche 16-29, bei dem der Gasanalysator einen Gasanalysator mit Gasentladungslampe umfasst.

31. Atemtest-Analysator gemäß einem der Ansprüche 16-30, bei dem der Gasanalysator eine Infrarotquelle umfasst, die ein diskontinuierliches Spektrum ausgibt.

32. Atemtest-Analysator gemäß einem der Ansprüche 16-31, bei dem der Analysator in Übereinstimmung mit Ergebnissen von laufenden Analysen der ersten und zweiten Proben feststellt, dass ein Test ein klinisch signifikantes Ergebnis liefert.

## Revendications

1. Procédé d'analyse de l'haleine comprenant :
la collecte d'au moins un premier échantillon d'au moins une première haleine expirée d'un sujet ;
la collecte d'au moins un second échantillon d'au moins une seconde haleine expirée du sujet ; et
l'analyse d'au moins lesdits premier échantillon et second échantillon pour y détecter des produits d'une substance marquée aux isotopes tandis que le sujet est couplé à un dispositif d'analyse de l'haleine ayant une sensibilité de détection auxdits produits de ladite substance marquée aux isotopes de l'ordre de quelques parties par million.

2. Procédé selon la revendication 1 et dans lequel ladite collecte d'au moins un premier échantillon, ladite collecte d'au moins un second échantillon et ladite analyse sont exécutées de manière continue sur plusieurs échantillons de l'haleine du sujet.

3. Procédé selon l'une quelconque des revendications précédentes, comprenant en outre l'étape consistant à traiter au moins une sortie de ladite analyse en temps réel.

4. Procédé selon l'une quelconque des revendications précédentes, dans lequel ladite analyse est effectuée en temps réel tandis que le sujet continue à fournir son haleine pour les analyses ultérieures.

5. Procédé selon l'une quelconque des revendications précédentes, dans lequel ladite analyse dudit premier échantillon et ladite analyse dudit second échantillon sont effectuées successivement après l'ingestion de ladite substance marquée aux isotopes par le sujet et avant la fin de la production des sous-produits de ladite substance marqués aux isotopes.

6. Procédé selon l'une quelconque des revendications précédentes, dans lequel ladite analyse est effectuée sur des haleines expirées individuelles.

7. Procédé selon l'une quelconque des revendications précédentes, dans lequel ladite collecte d'au moins un premier échantillon et ladite collecte d'au moins un second échantillon sont effectuées en continu.

8. Procédé selon l'une quelconque des revendications précédentes, dans lequel ladite analyse d'au moins un dudit premier échantillon et dudit second échantillon est effectuée sur un nombre accumulé d'échantillons d'haleine collectés du sujet.

9. Procédé selon l'une quelconque des revendications précédentes, dans lequel ladite collecte d'au moins un premier échantillon et ladite collecte d'au moins un second échantillon sont arrêtées automatiquement à un moment dans le temps déterminé par les résultats de ladite analyse.

10. Procédé selon l'une quelconque des revendications précédentes, dans lequel ladite analyse est arrêtée automatiquement à un moment dans le temps déterminé par les résultats de ladite analyse.

11. Procédé selon l'une quelconque des revendications précédentes, comprenant également la mesure d'un niveau d'échantillons de gaz marqués aux isotopes à intervalles réguliers.

12. Procédé selon l'une quelconque des revendications précédentes, dans lequel ladite analyse dudit premier échantillon et ladite analyse dudit second échantillon sont effectuées effectivement de manière quasi-continue.

13. Procédé selon l'une quelconque des revendications précédentes, dans lequel lesdits au moins un premier échantillon et au moins un second échantillon comprennent une multiplicité d'échantillons fréquents d'haleine collectée, de manière à ce que le point d'arrêt de l'analyse de l'haleine soit déterminé en fonction des résultats de ladite analyse obtenue en temps réel.

14. Procédé selon l'une quelconque des revendications précédentes, dans lequel lesdits au moins un premier échantillon et au moins un second échantillon sont collectés dans sensiblement chaque respiration successive, de sorte que le point d'arrêt de l'analyse de l'haleine est déterminé en fonction des résultats de ladite analyse obtenus en temps réel.

15. Procédé d'analyse de l'haleine selon l'une quelconque des revendications précédentes, dans lequel les résultats de ladite analyse dudit premier échantillon et dudit second échantillon sont utilisés pour déterminer si ladite analyse a un résultat significatif sur le plan clinique.

16. Appareil d'analyse de l'haleine comprenant :
une chambre d'analyse de l'haleine ;
un conduit d'admission de l'haleine destiné à transporter le gaz expiré d'un sujet à ladite chambre d'analyse de l'haleine ; et
un analyseur de gaz permettant d'analyser le gaz dans ladite chambre d'analyse de l'haleine,
ledit appareil d'analyse de l'haleine analysant au moins un premier échantillon collecté dans au moins une première haleine expirée du sujet et un second échantillon collecté dans au moins une seconde haleine expirée du sujet pour y détecter les produits d'une substance marquée aux isotopes tandis que le sujet est couplé audit appareil d'analyse de l'haleine, ledit analyseur de gaz ayant une sensibilité de détection pour lesdits produits de ladite substance marquée aux isotopes de l'ordre de quelques parts par million.

17. Appareil d'analyse de l'haleine selon la revendication 16, comprenant en outre une chambre de collecte de l'haleine.

18. Appareil d'analyse de l'haleine selon la revendication 17, dans lequel ladite chambre d'analyse de l'haleine et ladite chambre de collecte de l'haleine sont combinées, de sorte que ladite première et ladite seconde analyse se produisent dans ladite chambre de collecte de l'haleine.

19. Appareil d'analyse de l'haleine selon l'une quelconque des revendications 16 à 18, qui collecte et analyse en continu plusieurs échantillons de l'haleine du sujet et traite les sorties de ladite analyse en temps réel.

20. Appareil d'analyse de l'haleine selon l'une quelconque des revendications 16 à 19, dans lequel ladite analyse est effectuée en temps réel tandis que le sujet continue à fournir son haleine pour les analyses suivantes.

21. Appareil d'analyse de l'haleine selon l'une quelconque des revendications 16 à 19, dans lequel lesdites analyses sont effectuées successivement à des moments après l'ingestion de ladite substance marquée aux isotopes et avant la fin de la production des sous-produits de ladite substance marqués aux isotopes.

22. Appareil d'analyse de l'haleine selon l'une quelconque des revendications 16 à 21, qui effectue l'analyse sur des haleines expirées individuelles.

23. Appareil d'analyse de l'haleine selon l'une quelconque des revendications 16 à 22, qui procède à l'analyse de plusieurs échantillons de l'haleine du sujet collectés en continu dudit sujet.

24. Appareil d'analyse de l'haleine selon l'une quelconque des revendications 16 à 23, dans lequel la collecte desdits échantillons est arrêtée automatiquement à un moment dans le temps déterminé par les résultats de l'analyse desdits échantillons.

25. Appareil d'analyse de l'haleine selon l'une quelconque des revendications 16 à 24, dans lequel ladite analyse des échantillons est arrêtée automatiquement à un moment dans le temps déterminé par les résultats de ladite analyse desdits échantillons.

26. Appareil d'analyse de l'haleine selon l'une quelconque des revendications 16 à 25, qui mesure un niveau d'un échantillon de gaz marqué aux isotopes à intervalles réguliers.

27. Appareil d'analyse de l'haleine selon l'une quelconque des revendications 16 à 26, dans lequel les analyses de l'haleine expirée sont effectuées effectivement de manière quasi-continue.

28. Appareil d'analyse de l'haleine selon l'une quelconque des revendications 16 à 27, qui effectue plusieurs analyses sur sensiblement chaque respiration successive, permettant ainsi audit appareil de déterminer un point d'arrêt de la procédure d'analyse en fonction de résultats obtenus en temps réel.

29. Appareil d'analyse de l'haleine selon l'une quelconque des revendications 16 à 28, qui effectue plusieurs analyses sur des échantillons fréquents d'haleines collectées, permettant ainsi à l'appareil de déterminer un point d'arrêt d'une procédure d'analyse en fonction de résultats obtenus en temps réel.

30. Appareil d'analyse de l'haleine selon l'une quelconque des revendications 16 à 29, dans lequel ledit analyseur de gaz comprend un analyseur de gaz de tuyau d'évacuation des gaz.

31. Appareil d'analyse de l'haleine selon l'une quelconque des revendications 16 à 30, dans lequel ledit analyseur de gaz comprend une source infrarouge qui émet un spectre discontinu.

32. Appareil d'analyse de l'haleine selon l'une quelconque des revendications 16 à 31, dans lequel ledit appareil détermine qu'une analyse a un résultat significatif sur le plan clinique en fonction des résultats d'analyses en cours desdits premier et second échantillons.
